# EUROPEAN PATENT APPLICATION

(11) **EP 3 479 853 A1**
(43) Date of publication of application: **08.05.2019**
(21) Application number: 18204202.8
(22) Date of filing: 02.11.2018
(51) Int. Cl.: A61M 1/10

(54) **ENDOVASCULAR PULSATION BALLOON**

(30) Priority: 03.11.2017 US 201715802849
(71) Applicant: Cook Medical Technologies, LLC, Bloomington, IN 47404 (US)
(72) Inventor: Obermiller, F., West Lafayette, IN 47906 (US); Hadley, Richard, Otterbein, IN 47970 (US); Haselby, Kenneth, Battle Ground, IN 47920 (US); Kratzberg, Jarin, Lafayette, IN 47909 (US); Milner, Keith, West Lafayette, IN 47906 (US)
(74) Representative: Leach, Sean Adam

(57) **Abstract**

A vascular pulsation device may be provided including a pulsation portion and a reservoir portion. The pulsation portion may be insertable into a bodily passageway and may include an expandable segment, a first end, and a second end. The reservoir portion may be in fluid connection with the pulsation portion by a supply passage and a return passage. An opening of the supply passage may be positioned proximate to the second end of the pulsation portion. An opening of the return passage may be positioned proximate to the first end of the pulsation portion.

## Description

### Technical Field

This disclosure relates to medical devices and, in particular, to endovascular assemblies for improving vascular compliance of a vessel.

### Background

The statements in this section merely provide background information related to the present disclosure and may not constitute prior art.

Endovascular devices are commonly used in vascular passages when the vascular passageway becomes stiff and loses compliance. When a vascular passageway loses compliance due to, for example, age, congestive heart failure, or atherosclerosis, the vascular passageway stiffens and loses compliance, causing the heart to exert more force to effect the same volume of blood into the vascular passage. It is desirable to have an endovascular device which compensates for a lack of compliance of a vascular passage.

### Summary

Further areas of applicability will become apparent from the description provided herein. It should be understood that the description and specific examples are intended for purposes of illustration only and are not intended to limit the scope of the present disclosure.

In one embodiment, a vascular pulsation device is provided including a pulsation portion and a reservoir portion. The pulsation portion is insertable into a bodily passageway and includes an expandable segment, a first end, and a second end. The reservoir portion is in fluid connection with the pulsation portion by a supply passageway and a return passageway. The supply passageway includes an opening positioned proximate to the second end of the pulsation portion. The return passageway includes an opening positioned proximate to the first end of the pulsation portion.

In another embodiment, a vascular pulsation device is provided including a downstream portion and an upstream portion. The downstream portion includes an expandable segment and a reservoir. The expandable segment is sized to be insertable into an endovascular passageway. The expandable segment includes a first end, a second end. The reservoir is in fluid connection with the second end of the expandable segment by a supply passageway. The upstream portion includes a return passageway extending exclusively between the reservoir and the first end of the expandable segment.

In yet another embodiment, a method of operating a medical device to reduce cardiac back pressure is provided including inserting a pulsation potion into an endovascular passageway, fluidly connecting a reservoir portion to the pulsation portion, and creating a pressure gradient toward the reservoir portion. The pulsation portion includes an expandable segment arranged linearly between a first end and a second end of the pulsation portion. The reservoir portion is fluidly connected with the second end of the pulsation portion by a supply passageway. The reservoir portion is fluidly connected with the first end of the pulsation portion by a return passageway. The pressure gradient is created toward the reservoir portion by the supply passageway when an external force is applied to the expandable segment.

### Brief Description of the Drawings

The embodiments may be better understood with reference to the following drawings and description. The components in the figures are not necessarily to scale. Moreover, in the figures, like-referenced numerals designate corresponding parts throughout the different views.
FIG. 1 illustrates a cross-sectional view of an aortic passageway including a first example of a vascular pulsation device;
FIG. 2 illustrates a cross-sectional view of a second example of the vascular pulsation device;
FIG. 3 illustrates a cross-sectional view of a first example of a reservoir portion of the vascular pulsation device;
FIG. 4 illustrates a cross-sectional view of a second example of a reservoir portion of the vascular pulsation device;
FIG. 5 illustrates a view of a third example of the vascular pulsation device;
FIG. 6 illustrates a perspective view of a first example of a pulsation portion of the vascular pulsation device;
FIG. 7 illustrates a cross-sectional front view of the first example of a pulsation portion of the vascular pulsation device;
FIG. 8 illustrates a flow diagram of operations to operate a medical device to reduce cardiac back pressure.

The drawings described herein are for illustration purposes only and are not intended to limit the scope of the present disclosure in anyway.

### Detailed Description

The following description is merely exemplary in nature and is not intended to limit the present disclosure, application, or uses.

In some examples, a vascular pulsation device may be placed in the vascular passageway and manually inflated and deflated using a pump which coordinates inflation with the heartbeat of the patient. This configuration can be useful to assist the heart when the patient is stationary, such as when they are asleep or interred at a treatment facility. However, such an assembly cannot be used without the pumping assembly and therefore restricts the movement of the patient.

A vascular pulsation device, for example, is provided including a pulsation portion and a reservoir portion. The pulsation portion is insertable into a bodily passageway and includes an expandable segment, a first end, and a second end. The reservoir portion is in fluid connection with the pulsation portion by a supply passageway and a return passageway. The supply passageway includes an opening positioned proximate to the second end of the pulsation portion. The return passageway includes an opening positioned proximate to the first end of the pulsation portion.

One interesting feature of the systems and methods described below may be that the pulsation portion and reservoir portion are configured to provide a passive gradient of flow through the vascular pulsation device, passively working with the heartbeat of the patient to inflate and deflate the pulsation portion with the proper timing, thereby reducing stress on the heart muscles. Another interesting feature of the systems and methods described below may be that an active pump and heart sensors may not be needed to operate the vascular pulsation device. The lack of an active pump may grant increased freedom of movement to a patient utilizing the vascular pulsation device.

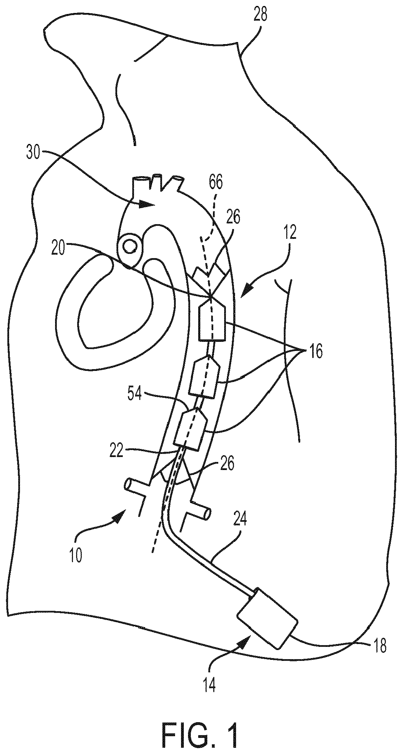

FIG. 1 illustrates a vascular pulsation device 10 positioned in a vascular passageway 30 of a patient 28. The vascular passageway 30 may be any passageway within the body of the patient 28 which is normally compliant to expansion and contraction under healthy conditions. Examples of the vascular passageway 30 may include the aorta, the common iliac arteries, or the subclavian arteries. The vascular pulsation device 10 may include a pulsation portion 12 and a reservoir portion 14. The pulsation portion 12 may be any portion of the vascular pulsation device 10 which inflates and deflates to control blood flow in the vascular passageway 30 and increase blood pressure within the vascular passageway 30. Examples of the pulsation portion 12 may include a balloon, a bladder, or a sac. The pulsation portion 12 is insertable into the vascular passageway 30 and may include an expandable segment 16, a first end 20, and a second end 22. As shown in FIG. 1, the first end 20 of the pulsation portion 12 may be positioned closer to the left ventricle of the heart (sometime described as being in a more "proximal", "upstream", or "cranial" position) compared to the second end 22. The second end 22 of the pulsation portion 12 may be positioned closer to the iliac arteries (sometimes described as being in a more "distal", "downstream", or "caudal" position) than the first end 20.

The expandable segment 16 may be any object which inflates and deflates to control blood flow in the vascular passageway 30 and increase blood pressure within the vascular passageway 30. Examples of the expandable segment 16 may include a balloon, a bladder, or a sac. As shown in FIG. 1, the pulsation portion 12 may include a more than one expandable segments 16. The expandable segments 16 may be arranged linearly within the vascular passageway 30 along a longitudinal axis 66 such that, when blood flows through the vascular passageway 30, the expandable segments 16 deflate sequentially from the first end 20 to the second end 22 of the pulsation portion 12. The expandable segment 16 may be made of any material which can inflate and deflate responsive to blood flow and prevent fluid from inside the expandable segment 16 from leaking into the vascular passageway 30, such as rubber or a polymer. The expandable segment 16 may be inflated with fluids such as saline, carbon dioxide or helium. Using a lower density fluid, such as helium, may increase the rate of flow of the fluid through the medical device 10, increasing the responsiveness of the expandable segment 16 to inflate or deflate in reaction to blood flow over the expandable segment 16. Using a higher density fluid, such as saline, may decrease the rate of flow of the fluid through the medical device 10, decreasing the responsiveness of the expandable segment 16 to inflate or deflate in reaction to blood flow over the expandable segment 16. The responsiveness of the expandable segment 16 to inflate or deflate may affect the back pressure of the blood within the vascular passageway 30.

Multiple expandable segments 16 may be coupled to one another by a tube 24 extending through at least one of the expandable segments. The tube 24 may be any object which is smaller than the fully inflated expandable segment 16 and which allows fluid flow between the pulsation portion 12 and the reservoir portion 14. The tube 24 may be made from a material such as rubber or a polymer. The ends of the expandable segments 16 may be coupled or attached to an outer surface of the tube 24. The ends of the expandable segment 16 may also include a tapered portion 54. The tapered portion 54 may be located at either the proximal end or distal end of the expandable segment 16 to improve blood flow over the expandable segment 16.

The pulsation portion 12 may also include a tether 26 which fixes the position of the pulsation portion 12 within the vascular passageway 30. The tether 26 may be any object which is expandable to grip the walls of the vascular passageway 30. Examples of the tether 26 may include a self-expanding stent portion or barbs. The tether 26 may be arranged anywhere along the length of the pulsation portion 12, such as, for example, the first end 20 and the second end 22. In some embodiments, the pulsation portion 12 may include a tether 26 proximate to the first end 20 of the pulsation portion 12 to prevent downstream movement of pulsation portion 12 as blood flows through the vascular passageway 30. In other embodiments, the pulsation portion 12 may include an additional tether 26 proximate the second end 22 of the pulsation portion 12 to better fix the position of the pulsation portion 12 within the vascular passageway 30.

The reservoir portion 14 may be any component capable of receiving and storing fluid outside of the pulsation portion 12. Examples of the reservoir portion 14 may include a tube, a sac, an inflatable balloon, or some other container. The reservoir portion 14 may receive fluid from the pulsation portion 12 from the tube 24 running between the pulsation portion 12 and the reservoir portion 14. The reservoir portion 14 may include a container 18 connected to the tube 24. When blood flow within the vascular passageway 30 deflates a portion of the pulsation portion 12, fluid within the pulsation portion 12 may flow into the container 18. As the fluid enters the container 18, the pressure within the container 18 may increase. The increased pressure within the container 18 may provide a passive force on the fluid to flow back into the pulsation portion 12. The container 18 may have a fixed volume or may be inflatable. The container 18 may be made of any material capable of retaining the fluid in the pulsation portion 12, such as rubber or a polymer. The container 18 and reservoir portion 14 may be located within the vascular passageway 30, inside the body of the patient 28 but outside the vascular passageway 30, or outside the body of the patient 28 altogether. In some embodiments, the tube 24 may run between the container 18 of the reservoir portion 14 and the second end 22 of the pulsation portion 12. In other embodiments, the tube 24 may run between the container 18 of the reservoir portion 14 and the first end 20 of the pulsation portion 12 to facilitate placement of the reservoir portion 14 in or outside of one of the right or left subclavian arteries.

The medical device 10, including pulsation portion 12 and the reservoir portion 14 may be deflated while being delivered to the vascular passageway 30. After implantation, the medical device 10 may be inflated to an operating volume. In some embodiments, the medical device 10 may only be partially inflated to allow compression of the expandable segment 16 to more easily allow air to flow from the pulsation portion 12 to the reservoir portion 14. The operating volume of the medical device 10 may be between about 50% to about 90% of the maximum volume of the medical device 10, and preferably between about 60% to about 80% of the maximum volume of the medical device 10.

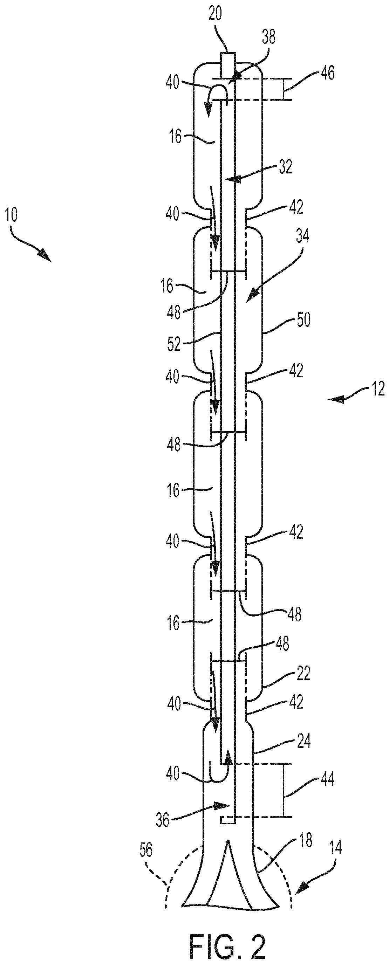

FIG. 2 illustrates another embodiment of the vascular pulsation device 10. As shown in FIG. 2, the reservoir portion 14 may be bifurcated in some embodiments, such that the pulsation portion 12 may be arranged within aorta of the patient 28 while the reservoir portion 14 may be arranged within the iliac arteries of the patient 28. The container 18 of the reservoir portion 14 may be inflatable to a maximum cross-sectional area 56 which is 50% to 90% of the cross-sectional area of the vascular passageway 30. Therefore, when the blood flowing over the pulsation portion 12 reaches the reservoir portion 14, the container 18 may deflate and force fluid back into the pulsation portion 12.

As shown in FIG. 2, the vascular pulsation device 10 may include a return passage 32 and a supply passage 34. The return passage 32 may be any conduit arranged to supply fluid from the reservoir portion to the first end 20 of the pulsation portion 12. Examples of the return passage 32 may include a tube, a channel, or a duct. The supply passage 34 may be any conduit arranged to supply fluid from the pulsation portion 12 to the reservoir portion 14. Examples of the supply passage 34 may include a tube, a channel, or a duct. As shown in FIG. 2, the supply passage 34 may be the interior of the expandable segments 16 in fluid connection by choked openings 42 arranged between the expandable segments 16. The return passage 32 may be positioned within the interior of the supply passage 34, as shown in FIG. 2, or may be positioned alongside the supply passage 34. The return passage 32 may be fluidly isolated from the supply passage 34 except at a return inlet 36 and a return outlet 38. The return outlet 38 may be located proximate to the first end 20 of the pulsation portion 12, for example, in the expandable segment 16 closest to the first end 20. The return outlet 38 may be positioned within the pulsation portion 12 in a location which is furthest from the reservoir portion 14. The return inlet 36 may be located in the reservoir portion 14 or as close as possible to the reservoir portion 14. The return inlet 36 may be located closer to the reservoir portion than the return outlet 38.

The return passage 32 and supply passage 34 may be arranged to bias the direction of flow 40 of the fluid within the pulsation portion. The direction of flow 40 through the supply passage 34 may be biased to flow toward the reservoir portion 14, or, as shown in FIG. 2, from the first end 20 of the pulsation portion 12 toward the second end 22. The direction of flow 40 through the return passage 32 may be biased to flow away from the reservoir portion 14, or, as shown in FIG. 2, from the second end 22 of the pulsation portion 12 toward the first end 20. As shown in FIG. 2, the direction of flow 40 through return passage 32 may be biased by varying the impedance to the flow across the vascular pulsation device. The impedance to flow may be highest at the return outlet 38 and decrease along the pulsation portion 12 approaching the reservoir portion 14. For example, a choked opening 42 fluidly connecting adjacent pairs of more proximally located expandable segments 16 may have a higher impedance to flow than a choked opening 42 fluidly connecting adjacent pairs of more distally located expandable segments 16. The impedance to flow through a choked opening 42 may be governed by the cross-sectional area 48 of the choked opening 42. Therefore, more proximally located choked openings may have a smaller cross-sectional area 48 than more distally located chocked openings 42. Similarly, the return outlet 38 may have greater impedance to flow than the return inlet 36 by the cross-sectional area 46 of the return outlet 38 being less than the cross-sectional area 44 than the return inlet 36. Flow may also be biased by the return passage 32 having greater impedance to flow than the supply passage 34.

When the heart ejects blood into the vascular passageway 30, it may be created in a wave defined by the contraction of the heart. When the wave of blood flow passes over the most proximal expandable segment 16 of the pulsation portion 12, the expandable segment 16 may be partially or fully deflated. From this compression a majority of the fluid in the expandable segment 16 may flow out of the expandable segment 16 through the choked opening 42, which has less impedance to flow than the other opening, the return outlet 38. As the wave continue to deflate more distally located expandable segments, 16, the flow of the fluid moving out of the expandable segments 16 may be biased toward the further distally located choked openings 42 with even less impedance to flow, and ultimately, into the return inlet 36, which may have less impedance to flow than any of the choked openings. As the wave passes over then distal-most expandable segment 16, the majority of fluid in the pulsation portion has flowed into the return passage 32. As the pressure within the return passage rises, the fluid may exit through return outlet 38 and reinflate the more proximal expandable segments 16 over which the wave has already passed.

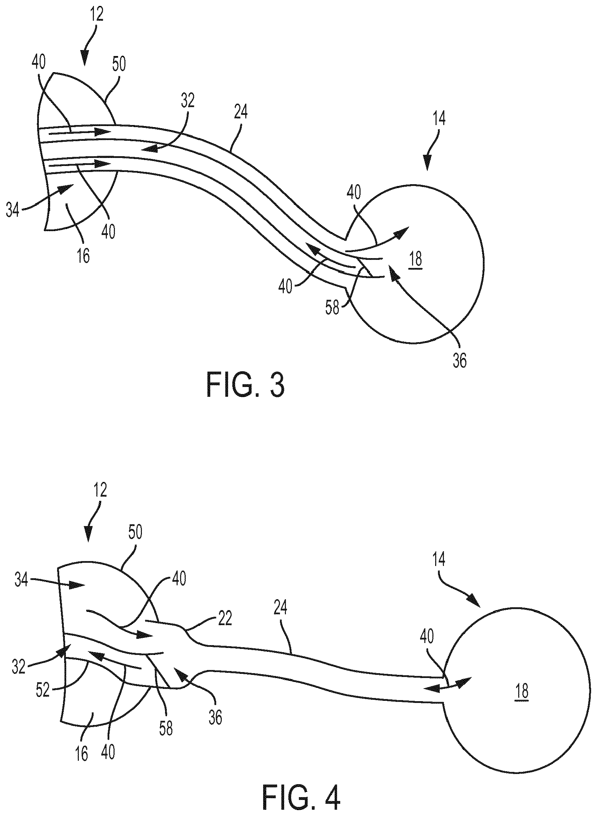

FIG. 3 illustrates a cross-sectional view of an example of the reservoir portion 14. As shown in FIG. 3, in some embodiments, both the supply passage 34 and the return passage 32 may extend through the tube 24 and into the container 18. In such an arrangement, fluid exiting the expandable segments 16 due to deflation travels to the container 18 to enter the return passage 32 and be reintroduced to the pulsation portion 12. This arrangement may be useful where the container 18 of the reservoir portion 18 is located outside the vascular passageway 30.

The return inlet 36 may include a check valve 58 to bias the direction of flow 40 of the fluid. The check valve 58 may be any device which allows fluid to flow into the return passage 32 through the return inlet 36 but prevents fluid from flowing out of the return passage 32 through the return inlet 36. Examples of the check valve 58 may include a swing check valve, a lift check valve, or a wafer check valve. The resistance of the check valve 68 may be adjusted to define the impedance of the return inlet 36. In some arrangements, the check valve 58 may, instead be arranged in the supply passage 34, to prevent fluid from flowing toward the most proximally located expandable segment 16 by the supply passage 34.

FIG. 4 illustrates an alternative embodiment of the reservoir portion 14. As illustrated in FIG. 4, the return passage 32 and the supply passage 34 may end proximate to the second end 22 of the pulsation portion 12. The tube 24 connecting the pulsation portion 12 to the container 18 may have a smaller diameter than if the return passage 32 and the supply passage 34 extended through the tube 24, as shown in FIG. 3. In such an arrangement, fluid exiting the supply passage 34 may flow into the reservoir portion 14, or may flow into the return inlet 36 of the return passage 32. In such an arrangement, the reservoir portion 14, may provide a back pressure to the fluid moving through the pulsation portion 12 which is responsive to the deflation and inflation of the expandable segments 16 of the pulsation portion 12.

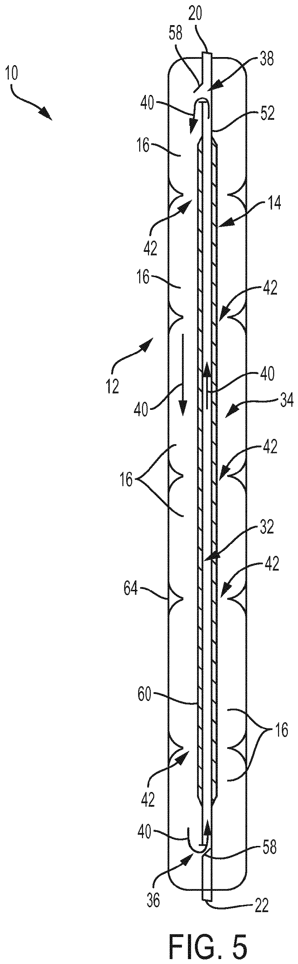

FIG. 5 illustrates an embodiment of the vascular pulsation device 10 including a reservoir portion 14 which is positioned inside the pulsation portion 12. In some embodiments, the reservoir portion 14 may include the return passage 32 which is positioned within the supply passage 34 of the pulsation portion 12. In such an embodiment, the external container 18 shown in FIGS. 1-4 may be unnecessary, as fluid flowing from the deflated expandable segments 16 may flow directly into the return passage 32 through the return inlet 36.

As illustrated in FIG. 5, the return passage 32 may include a check valve 58 at both the return inlet 36 and the return outlet 38 to bias the direction of flow 40 in the distal direction through the supply passage 34 and in a proximal direction through the return passage 32. The resistance of the check valves 58 may be adjusted to define the impedance through both of the return inlet 36 and the return outlet 38. The direction of flow 40 may be further biased as described above by the return outlet 38 having a greater impedance than the return inlet 36.

In such an embodiment as illustrated in FIG. 5, the return passage 32 include an expandable wall 60 which may expand as fluid flows into the return passage 32. The expandable wall 60 may be made of materials such as rubber or a polymer. The expandable wall 60 may have a greater rigidity than a walls 50 of the expandable segments 16. Therefore, deflation of an expandable segment 16 may cause an inflation of the return passage 32 which is less than the volume of the deflation of the expandable segment 16.

In some embodiments, the pulsation portion 12 may include a sheath 64 coupled to the expandable segments 16 and extending across and between expandable segments 16. The sheath 64 may be made of the same material as the wall 50 of the expandable segment 16, such as rubber or a polymer. As the individual expandable segments 16 inflated and deflate, the sheath 64 may maintain a smooth curvature on the outer surface of the pulsation portion 12 allowing blood to flow with less resistance in the vascular passageway 30.

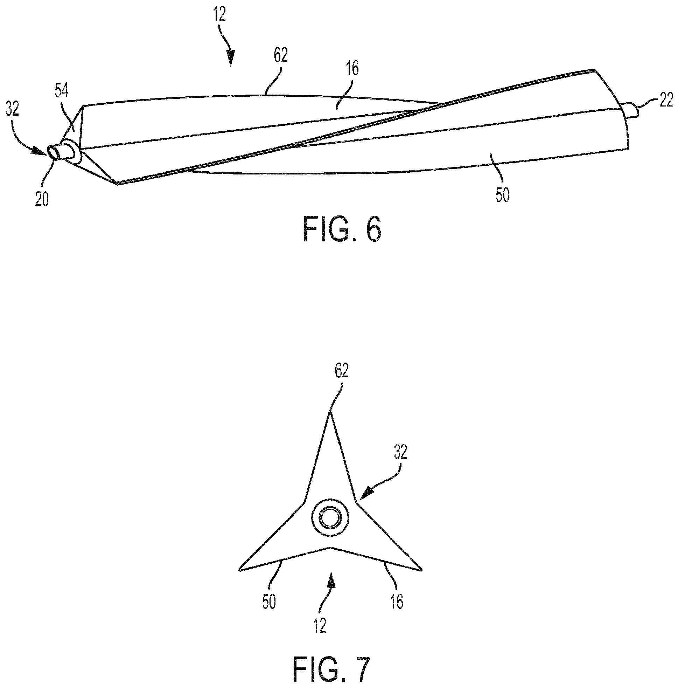

FIGS. 6 and 7 illustrate a possible embodiment of the expandable segment 16 of the pulsation portion 12. In some embodiments, the expandable segments 16 may be cylindrical, but as illustrated in FIGS. 6 and 7, the expandable segments 16 may also have a more complex shape. For example, the wall 50 of the expandable segments 16 may be made into a triangular cross-sectional shape. A triangular shape may be ideal to allow edges 62 of the triangular expandable segment 16 to rest against the walls of the vascular passageway 30. If all three edges 62 rested against the walls of the vascular passageway 30, the expandable segment 16 would be naturally centered within the vascular passageway 30, while optimizing the cross-sectional area of the expandable segment 16. Furthermore, the walls 50 expandable segment 16 may include a helical twist along the length of the expandable segment 16. Such a twist may improve the flow of blood over the expandable segment 16.

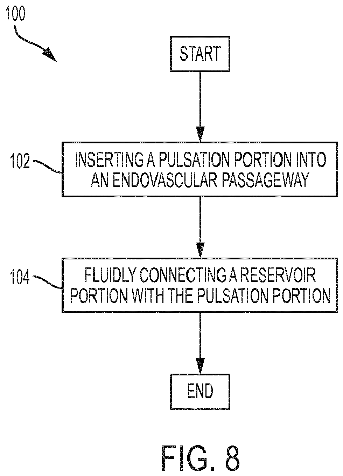

FIG. 8 illustrates a flow diagram of operations to utilize a medical device to reduce cardiac back pressure. The operations (100) may include fewer, additional, or different operations than illustrated in FIG. 8. Alternatively, or in addition, the operations (100) may be performed in a different order than illustrated.

Initially, the method of operations (100) includes inserting a pulsation portion 12 into an endovascular passageway 30 (102). The pulsation portion 12 may include a plurality of expandable segments 16 arranged linearly between the first end 20 and the second end 22 of the pulsation portion 12. The method of operations (100) also may include fluidly connecting a reservoir portion 14 with the pulsation portion 12 (104). The supply passage 34 of the pulsation portion 12 may be fluidly connected to the reservoir portion 14 at the second end 22 of the pulsation portion 12. The return passage 32 of the pulsation portion 12 may be fluidly connected to the reservoir portion 14 at the first end 20 of the pulsation portion 12. These steps (102, 104) may be done concurrently or separately.

Described herein is a method of operating a medical device (10) to reduce cardiac back pressure, the method comprising:
inserting a pulsation portion (12) of the medical device (10) into an endovascular passage (30), the pulsation portion (12) comprising a plurality of expandable segments (16) arranged linearly between a first end (20) and a second end (22) of the pulsation portion (12);
fluidly connecting a reservoir portion (14) with the second end (22) of the pulsation portion (12) by a supply passage (34) and with the first end (20) of the pulsation portion (12) by a return passage (32); and
creating a pressure gradient toward the reservoir portion (14) by the supply passage (34) when an external force is applied to one of the plurality of expandable segments (16). In this method, the pressure gradient may be maintained by a first check valve (58) positioned in the return passage (32) and a second check valve (58) positioned in the supply passage (34). The pressure gradient may be maintained by the return passage (32) having an impedance to fluid flow which is greater than an impedance of the supply passage (34).

In addition to the advantages that have been described, it is also possible that there are still other advantages that are not currently recognized but which may become apparent at a later time. While various embodiments have been described, it will be apparent to those of ordinary skill in the art that many more embodiments and implementations are possible. Accordingly, the embodiments described herein are examples, not the only possible embodiments and implementations.

A vascular pulsation device may be provided including a pulsation portion and a reservoir portion. The pulsation portion may be insertable into a bodily passageway and may include an expandable segment, a first end, and a second end. The reservoir portion may be in fluid connection with the pulsation portion by a supply passage and a return passage. An opening of the supply passage may be positioned proximate to the second end of the pulsation portion. An opening of the return passage may be positioned proximate to the first end of the pulsation portion.

To the extent that the methods described herein may be applied to tissues of the human or animal body, it will be appreciated that such methods may not provide any surgical or therapeutic effect. For example, such methods may be applied ex vivo, to tissue samples that are not part of the living human or animal body. In addition, the methods described herein may be practiced on meat, tissue samples, cadavers, and other non-living objects. Their implementation on cadavers may find particular utility in the training of surgeons and medical professionals.

## Claims

1. A vascular pulsation device (10), comprising:
a pulsation portion (12) being insertable into a bodily passageway (30), the pulsation portion (12) comprising an expandable segment (16), a first end (20) and a second end (22); and
a reservoir portion (14) in fluid connection with the pulsation portion (12) by a supply passage (34) and by a return passage (32), a return inlet (36) of the supply passage (34) being positioned proximate to the second end (22) of the pulsation portion (12) and a return outlet (38) of the return passage (32) being positioned proximate to the first end (20) of the pulsation portion (12).

2. The vascular pulsation device (10) of claim 1, wherein the return passage (32) and the supply passage (34) are arranged to create a pressure gradient toward the reservoir portion (14) by the supply passage (34) when an external force is sequentially applied from the first end (20) of the pulsation portion (12) to the second end (22) of the pulsation portion (12).

3. The vascular pulsation device (10) of claims 1 through 2, wherein the pulsation portion (12) comprises a plurality of expandable segments (16) arranged linearly between the first end (20) and the second end (22) of the pulsation portion (12), each of the plurality of expandable segments (16) being in fluid connection with another of the plurality of expandable segments (16).

4. The vascular pulsation device (10) of claim 3, wherein each of the expandable segments (16) has a tapered first end (54).

5. The vascular pulsation device (10) of claims 3 through 4, wherein the pulsation portion (12) further comprises a sheath (64) coupled to the plurality of expandable segments (16) such that inflation of one of the plurality of expandable segments (16) creates a smooth curvature on an outer surface of the sheath (64).

6. The vascular pulsation device (10) of claims 1 through 5, wherein the reservoir portion (14) is spaced apart from the pulsation portion (12).

7. The vascular pulsation device (10) of claims 1 through 5, wherein the reservoir portion (14) is positioned within an interior of the pulsation portion (12).

8. The vascular pulsation device (10) of claim 7, further comprising a first check valve (58) positioned between the first end (20) of the pulsation portion (12) and the reservoir portion (14), wherein the first check valve (58) prevents flow from the first end (20) of the pulsation portion (12) to the reservoir portion (14) through the return passage (32), for example further comprising a second check valve (58) positioned between the second end (22) of the pulsation portion (12) and the reservoir portion (14), wherein the second check valve (58) prevents flow from the second end (22) of the pulsation portion (12) to the reservoir portion (14) through the supply passage (34), and wherein the first check valve (58) has an impedance which is greater than an impedance of the second check valve (58).

9. An vascular pulsation device (10), comprising:
a downstream portion comprising an expandable segment (16) sized to be insertable into an endovascular passage (30), the expandable segment (16) comprising a first end (20) and a second end (22), and a reservoir (18) in fluid connection with the second end (22) of the expandable segment (16) by a supply passage (34); and
an upstream portion comprising a return passage (32) extending exclusively between the reservoir (18) and the first end (20) of the expandable segment (16).

10. The vascular pulsation device (10) of claim 9, wherein the reservoir (18) is implantable outside the endovascular passage (30).

11. The vascular pulsation device (10) of claims 9 through 10, wherein the return passage (32) comprises a first check valve (58) configured to prevent fluid flow from the first end (20) of the expandable segment (16) to the reservoir (18) through the return passage (32); and the supply passage (34) comprises a second check valve (58) to prevent fluid flow from the second end (22) of the expandable segment (12) to the reservoir (18) through the supply passage (34).

12. The vascular pulsation device (10) of claims 9 through 11, wherein an impedance of the return passage (32) is greater than an impedance of the supply passage (34).

13. The vascular pulsation device (10) of claim 12, wherein the downstream portion comprises a plurality of expandable segments (16) arranged along an axis (66) from a first end of the downstream portion to a second end of the downstream, wherein the return passage (32) comprises an return outlet (38) into one of the plurality of expandable segments (16) positioned closest to the first end of the downstream portion, for example wherein the return passage (32) comprises a return inlet (36) in one of the plurality of expandable segments (16) positioned closest to the second end of the downstream portion, and the supply passage (34) comprises a plurality of openings (42), each of the plurality of openings (42) positioned between an adjacent pair of the plurality of expandable segments (16), wherein the impedance of the plurality of openings (42) in the supply passage (34) decreases from the first end of the downstream portion to the second end of the downstream portion.

14. The vascular pulsation device (10) of claims 9 through 13, wherein the expandable segment (16) comprises an outer surface (50) having a helical shape.

15. The vascular pulsation device (10) of claims 9 through 14, wherein the expandable segment (16) has a triangular cross-sectional shape.
